# EUROPEAN PATENT SPECIFICATION

(11) **EP 3 508 856 B1**
(45) Date of publication and mention of the grant of the patent: **24.06.2026**
(21) Application number: 17845449.2
(22) Date of filing: 30.08.2017
(51) Int. Cl.: G01N 33/68, G01N 33/576

(54) **NOVEL LIVER CIRRHOSIS OR LIVER FIBROSIS MARKER**
NEUARTIGER LEBERZIRRHOSE- ODER LEBERFIBROSEMARKER
NOUVEAU MARQUEUR DE LA CIRRHOSE DU FOIE OU DE LA FIBROSE DU FOIE

(30) Priority: 31.08.2016 CN 201610798291
(43) Date of publication of application: 10.07.2019
(73) Proprietor: Lu, Fengmin, Beijing 100191 (CN); Yao, Mingjie, Beijing 100191 (CN)
(72) Inventor: LU, Fengmin, Beijing 100191 (CN); YAO, Mingjie, Beijing 100191 (CN); ZHAO, Jingmin, Beijing 100039 (CN)
(74) Representative: Ipsilon
(86) International application number: PCT/CN2017/099714
(87) International publication number: WO 2018/041147

(56) References cited:
- WO-A2-2005/026687
- WO-A2-2005/026687
- WO-A2-2008/060394
- CN-A- 101 328 214
- CN-A- 101 328 214
- CN-A- 101 735 319
- CN-A- 101 735 319
- CN-A- 103 604 918
- CN-A- 103 604 918
- CN-A- 105 189 549
- CN-A- 105 189 549
- CN-A- 106 405 104
- US-A1- 2014 134 606
- US-A1- 2014 134 606
- QIAO YONG ET AL: "Serum gp73 is also a biomarker for diagnosing cirrhosis in population with chronic HBV infection", CLINICAL BIOCHEMISTRY, ELSEVIER INC, US, CA, vol. 47, no. 16, 25 August 2014 (2014-08-25), pages 216 - 222, XP029091575, ISSN: 0009-9120, DOI: 10.1016/J.CLINBIOCHEM.2014.08.010
- TAN Q EN,GUO WEI,ZHANG CHUNYAN, JIONG ET AL: "Value of Golgi protein 73(GP73) for diagnosis and grading of liver cirrhosis", ZHENDUANXUE LILUN YU SHIJIAN -JOURNAL OF DIAGNOSTICS CONCEPTS & PRACTICE, SHANGHAI RUIJIN YIYUAN, CN, vol. 12, no. 5, 31 December 2013 (2013-12-31), pages 516 - 521, XP009513624, ISSN: 1671-2870
- LU , FENGMIN: "Serological diagnosis of hepatocellular carcinoma: challenges and opportunities", JOURNAL OF CLINICAL HEPATOLOGY, vol. 33, no. 7, 31 July 2017 (2017-07-31), pages 1262 - 1265, XP009513598, ISSN: 1001-5256, DOI: 10.3969/j.issn.1001-5256.2017.07.011
- QIAO, Y. ET AL.: "Serum gp73 is also a biomarker for diagnosing cirrhosis in population with chronic HBV infection", CLINICAL BIOCHEMISTRY, vol. 47, 25 August 2014 (2014-08-25), pages 216 - 222, XP029091575, DOI: doi:10.1016/j.clinbiochem.2014.08.010
- TAN, QIWEN ET AL.: "Value of Golgi protein 73(GP73) for diagnosis and grading of liver cirrhosis", JOURNAL OF DIAGNOSTICS CONCEPTS & PRACTICE, vol. 12, no. 5, 31 December 2013 (2013-12-31), pages 516 - 521, XP009513624, ISSN: 1671-2870

## Description

### TECHNICAL FIELD

The invention belongs to the field of biomedicine, and relates to a novel use of Golgi protein 73 and/or Golgi protein 73 antibody in the in vitro/ ex vivo diagnosis/prognosis of liver diseases as well as to the use of the respective kits.

### BACKGROUND

It has been 16 years since Kladney et al. discovered Golgi 73 (GP73) in the study of adult giant cell hepatitis in the year of 2000 (Kladney RD, Bulla GA, Guo L, et al. Gene. 2000. 249(1-2): 53-65). GP73, also named as Golgiphosphoprotein 2 (Golph2) and Golgi membraneprotein 1 (Golm1), is a transmembrane glycoprotein consisting of 402 amino acids and having a relative Mw of 73 kDa. It is usually located in the trans-Golgi complex. The gene encoding GP73 protein locates on chromosome 9 and the total length is 3090 nucleotides, with the encoding region locates on 199-1404 nt. GP73 is expressed in various tissues with varied expression level. It is highly expressed in small intestine, colon and stomach, and weakly expressed in liver, kidney, spleen, lung, uterus and testis, with the lowest expression level in heart. In liver tissue of normal human, GP73 is mainly expressed in the bile duct epithelial cells, but barely in the normal liver cells. Kladney et al. found in subsequent studies that the expression level of GP73 in tissues of patients with viral (HBV and HCV) infection and non-viral (alcoholic liver disease and autoimmune hepatitis) liver diseases (chronic hepatitis B, chronic hepatitis C, alcoholic cirrhosis and cirrhosis caused by autoimmune hepatitis) were significantly increased, which was 70 folds higher than those in normal liver tissues, however, there was no significant difference in between the liver disease groups. Further studies by Kladney et al. showed that GP73 expression was low in normal liver tissues. GP73 expression level was significantly increased in liver diseases caused by various factors (viral correlation, alcoholism or autoimmunity), while in the meantime, the expression of GP73 in bile duct epithelial cells did not change significantly, however, the expression of GP73 in damaged hepatocytes was significantly higher than that in pre-disease hepatocytes, indicating that the high expression of GP73 is a consequence of up-regulation of GP73 expression in hepatocytes, which is the pathological basis of the use of GP73 in the field of liver diseases (Kladney RD, Cui X, Bulla GA, Brunt EM, Fimmel CJ. Hepatology. 2002. 35(6): 1431-40.). Iftikhar et al. found that the expression of GP73 was increased in acute hepatitis (viral and autoimmune) and progressive cirrhosis (alcoholic liver disease and chronic hepatitis C). After immunosuppressive treatment of autoimmune hepatitis, the expression of GP73 was decreased to normal level, indicating that the initial high expression of GP73 in the disease could be reversed. Iftikhar believes that there could be two regulatory mechanisms present in the expression of GP73: one triggered during acute hepatocellular injury, the other during the progression of chronic liver disease (Iftikhar R, Kladney RD, Havlioglu N, et al. Am J Gastroenterol. 2004. 99(6): 1087-95.). Block el al. made an important step in the use of GP73 in the clinical field of hepatocellular carcinoma in 2005. When they studied the relationship between the expressions of GP73 in serum of woodchuck, they found that woodchucks suffered from hepatocellular carcinoma have dramatically higher concentrations of GP73, as compared with woodchucks without a diagnosis of HCC. The same conclusion was reached when they studied the expression of GP73 in human hepatocellular carcinoma, in which the sensitivity and specificity of GP73 in the diagnosis of hepatocellular carcinoma were 69% and 75%, suggesting that the level of GP73 in serum of patients with hepatocellular carcinoma was significantly increased, and GP73 might be better than AFP in the diagnosis of early hepatocellular carcinoma (Block TM, Comunale MA, Lowman M, et al. Proc Natl Acad Sci U S A. 2005. 102(3): 779-84.). Cassandra therefore suggested that GP73 might be a better serum marker for the diagnosis of hepatocellular carcinoma, especially early hepatocellular carcinoma.

Marrero (Marrero JA, Romano PR, Nikolaeva O, et al. J Hepatol. 2005. 43(6): 1007-12.) and Hu (Hu JS, Wu DW, Liang S, Miao XY. Med Oncol. 2010. 27(2): 339-45.) et al. studied GP73 in human hepatocellular carcinoma earlier. They believed that GP73 had significantly higher level in hepatocellular carcinoma than that in patients with hepatitis and cirrhosis, and it was better than AFP in the diagnosis of hepatocellular carcinoma. Yilei Mao et al. (Mao Y, Yang H, Xu H, et al. Gut. 2010. 59(12): 1687-93.) had in-depth clinical study on GP73, and their 4217 large-sample clinical trial showed that: when GP73 with relative unit 8.5 was set as the critical value for hepatocellular carcinoma diagnosis, the diagnostic sensitivity and specificity were 74.6% and 97.4% respectively, which were higher than that in AFP detection. Combination diagnosis of GP73 and AFP showed a better effect. In the meantime, the study showed that although GP73 level was elevated with different degrees in liver benign diseases (such as hepatitis, cirrhosis) and other malignant tumors outside the liver, it was particularly prominent in the hepatocellular carcinoma. The research results of Mao et al. greatly promoted the use of GP73 in the field of hepatocellular carcinoma diagnosis. The advantage of GP73 over AFP is that it improves the detection of some AFP-negative hepatocellular carcinoma patients.

The role of GP73 in predicting the prognosis of hepatocellular carcinoma is also worth discussing. The results of Sun et al. (Sun Y, Yang H, Mao Y, et al. J Gastroenterol Hepatol. 2011. 26(7): 1207-12.) showed that the serum GP73 concentration was not related to age, sex, number of tumors, liver function, size of tumors or portal vein invasion of patients, but the GP73 concentration in tumor tissue was closely related to the diameter of tumors, portal vein invasion and degree of tumor differentiation, suggesting that the concentration of GP73 in tissues is related to the malignant degree of tumors, but it fails to reveal the relationship between GP73 and patient survival.

The role of GP73 in evaluating the efficacy of cancer treatment also requires our attention. Harm et al. (Hann HW, Wang M, Hafner J, et al. Cancer Biomark. 2010. 7(6): 269-73.) believe that patients with decreased GP73 concentration after first treatment can achieve nearly 6 years of tumor-free survival, while patients with increased GP73 concentration after first treatment are more likely to have recurrence within 5 years. Mao et al. also investigated the relationship between serum expression level of GP73 and tumor recurrence after hepatocellular carcinoma surgery. The results showed that the postoperative GP73 concentration decreased progressively, peaked at the 14^{th} day after surgery, and the GP73 concentration increased significantly again when the disease relapsed. The above studies suggest that GP73 may play a role in postoperative follow-up and monitoring of disease recurrence in patients with hepatocellular carcinoma.

In WO2005/026687 subjects with chronic hepatitis B and C virus infections were evaluated and GP73 was found to be a suitable marker for hepatocarcinoma, while no statistically significant changes were found in precancerous stages.

At present, cumulative literatures have shown that GP73 can be used as a serum marker for the diagnosis of hepatocellular carcinoma, and combination detection of GP73 and AFP can improve the sensitivity and specificity of diagnosis. Previous studies have shown that GP73 may play a role in the monitoring of hepatocellular carcinoma after operation. However, in fact, there are different opinions on the role of GP73 in hepatocellular carcinoma. Morota (Morota K, Nakagawa M, Sekiya R, et al. Clin Chem Lab Med. 2011. 49(4): 711-8.) and Gu (Morota K, Nakagawa M, Sekiya R, et al. Clin Chem Lab Med. 2011. 49(4): 711-8.) et al. believe that GP73 can be used as a diagnostic marker for liver diseases, and its concentration in cirrhosis and hepatocellular carcinoma patients is significantly higher than that of hepatitis patients and healthy subjects, but it is not recommended as a diagnostic marker for hepatocellular carcinoma alone. Yamamoto et al. (Yamamoto K, Imamura H, Matsuyama Y, et al. J Gastroenterol. 2010. 45(12): 1272-82.) showed that GP73 concentration did not change significantly before and after surgery, so it is not recommended as a clinical indicator for post-operative detection. Meanwhile, the results published previously are mostly from retrospective clinical studies. Therefore, there is still a need for higher-level evidence obtained from large-scale, prospective and multi-center cooperation to support whether GP73 really has diagnostic value for hepatocellular carcinoma or not. In addition, although the expression of GP73 is elevated in some liver diseases such as acute hepatitis (viral and autoimmune) and progressive cirrhosis (alcoholic liver disease and chronic hepatitis C), there is no statistically significant difference between the two groups. Whether GP73 can be used as a diagnostic marker for pre-hepatocellular carcinoma conditions remains to be revealed.

### SUMMARY OF THE INVENTION

The purpose of the present invention is to provide a new use of a substance for detecting the level of Golgi protein 73.

The present invention is defined in its broadest sense by the appended claims.

In the present invention, Golgi protein 73 (GP73) can be used as a marker for diagnosis/prognosis of liver diseases, the liver disease being selected from non-hepatitis B virus infection and the diagnosis/prognosis is distinguishing between significant liver fibrosis, severe liver fibrosis and early cirrhosis, the results are accurate and easy to operate. Meanwhile, according to the understanding of the prior art, markers are generally used for the diagnosis, treatment and prognosis of diseases; the change and good discrimination of GP73 in different disease degree groups also fully demonstrate that those skilled in the art can use GP73 as a marker for adjuvant treatment or prognosis judgment of liver diseases (i. e. non-hepatitis B virus infected liver diseases) based on the experimental results of the present invention.

In the use described above, the substance of detecting the Golgi protein 73 level comprises Golgi protein 73 and/or Golgi protein 73 antibody;
The Golgi protein 73 is human Golgi protein 73, and the antibody against Golgi protein 73 is the antibody against human Golgi protein 73;
The human Golgi protein 73 antibody is a monoclonal antibody, a polyclonal antibody or a genetically engineered human Golgi protein 73 antibody.

In any one of the use described above, the substance of detecting Golgi protein 73 level further comprises a reagent and a required instrument for detecting the Golgi protein 73 level besides Golgi protein 73 and/or the Golgi protein 73 antibody.

Use of recombinant vector, expression cassette, transgenic cell line or recombinant strain containing Golgi protein 73 encoding gene as a biomaterial of preparing Golgi protein 73 for preparing the products for screening, auxiliarily diagnosing liver diseases or auxiliarily determining the prognosis of liver diseases is described herein.

Use of recombinant vector, expression cassette, transgenic cell line, recombinant strain containing Golgi protein 73 antibody encoding gene or immunogen or antigen of Golgi protein 73 antibody as a biomaterial of preparing Golgi protein 73 antibody for preparing the products for screening, auxiliarily diagnosing liver diseases or auxiliarily determining the prognosis of liver diseases is also described herein.

In any one of the use described above, the disease is cirrhosis or liver fibrosis, and the cirrhosis or liver fibrosis is cirrhosis or the liver fibrosis resulting from non-hepatitis B virus infection.

In any one of the use described above, subject in the screening or diagnosing by the product is a patient with cirrhosis, liver fibrosis or a healthy person.

In any one of the use described above, the product is tested in serum, plasma or interstitial fluid, and preferably human serum, human plasma or human interstitial fluid.

In a further aspect, thee present invention relates to the use of a kit in the in vitro/ex vivo diagnosis/prognosis of liver diseases, the kit comprising a substance of detecting Golgi protein 73 level in a sample, the liver disease being selected from a liver disease resulting from non-hepatitis B virus infection; and the diagnosis/ prognosis being distinguishing between significant liver fibrosis (F > 2) , severe liver fibrosis (F > 3) and early cirrhosis (F = 4), wherein the sample is human serum, human plasma or human interstitial fluid, wherein the substance of detecting Golgi protein 73 level in a sample is Golgi protein 73 and/or Golgi protein 73 antibody.

In any of the kits described above, detection methods by the kit include enzyme-linked immunosorbent assay, chemiluminescence, electrochemiluminescence, enzymatic chemiluminescence, time-resolved fluorescence, or upconversion luminescence.

### BENEFICIAL EFFECT OF THE INVENTION

The beneficial effect of the invention is that the GP73 has a relatively modest diagnostic value for hepatocellular carcinoma, and cannot distinguish the patients with hepatocellular carcinoma from those with hepatitis and cirrhosis, and its discrimination ability is not as good as that of the AFP. On the contrary, the sensitivity of GP73 in diagnosing cirrhosis is higher than that of serum AFP, and its ability in distinguishing patients with hepatitis or cirrhosis is stronger.

### BRIEF DESCRIPTION OF THE DRAWINGS

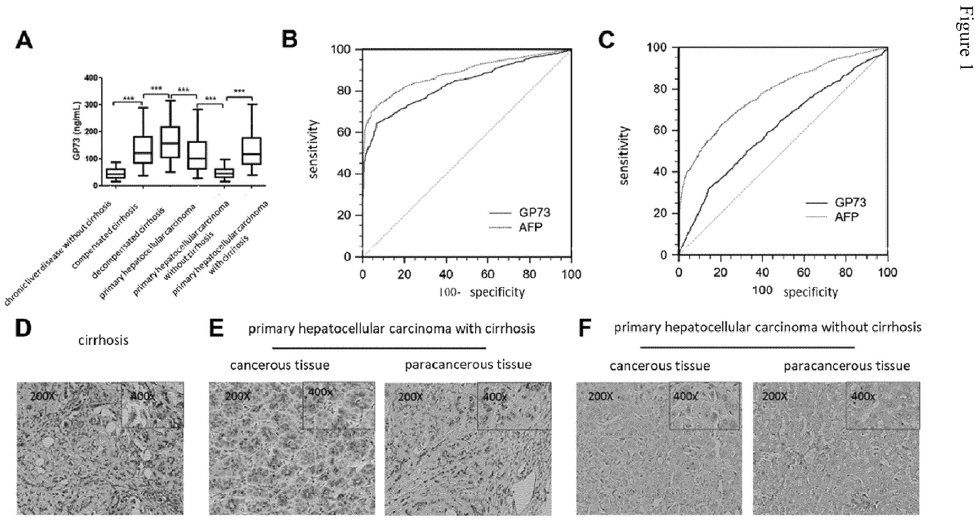

Fig. 1 shows the ROC curve of the diagnostic value of GP73 for hepatocellular carcinoma. (A) GP73 concentrations were measured in non-cirrhosis chronic liver disease group, cirrhosis group, hepatocellular carcinoma (HCC) group, non-cirrhosis HCC group and HCC group transformed from cirrhosis. (B) The sensitivity in distinguishing HCC from non-cirrhosis chronic liver disease. (C) The sensitivity in distinguishing HCC from cirrhosis. (D)-(E) Immunohistochemical images of liver in different degrees of disease.

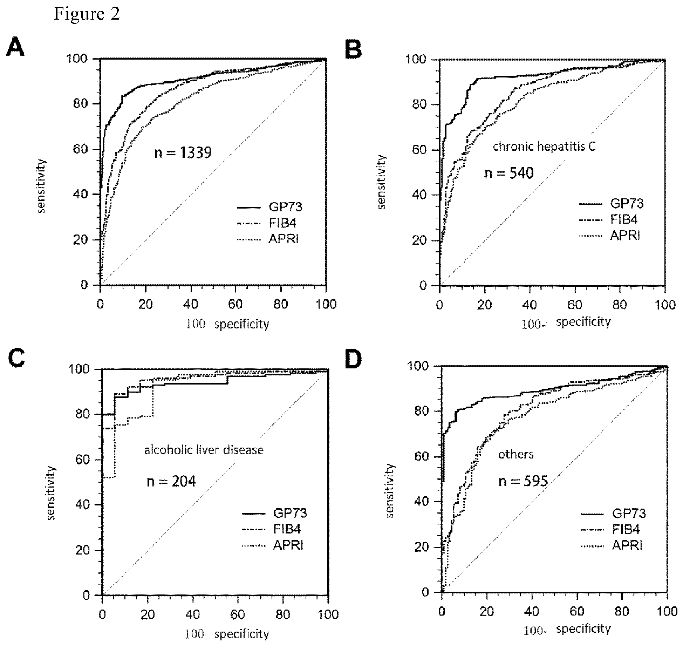

Fig. 2 shows the ROC curve of the diagnostic value of GP73 for cirrhosis. (A) The diagnostic value of GP73 in recruited patients with cirrhosis. (B) ROC curves of different markers in chronic hepatitis C (CHC). (C) ROC curves of different markers in chronic alcoholic liver disease (ALD). (D) The diagnostic value of GP73 for cirrhosis in all other populations (autoimmune liver disease, nonalcoholic fatty liver disease and primary biliary cirrhosis, etc.).

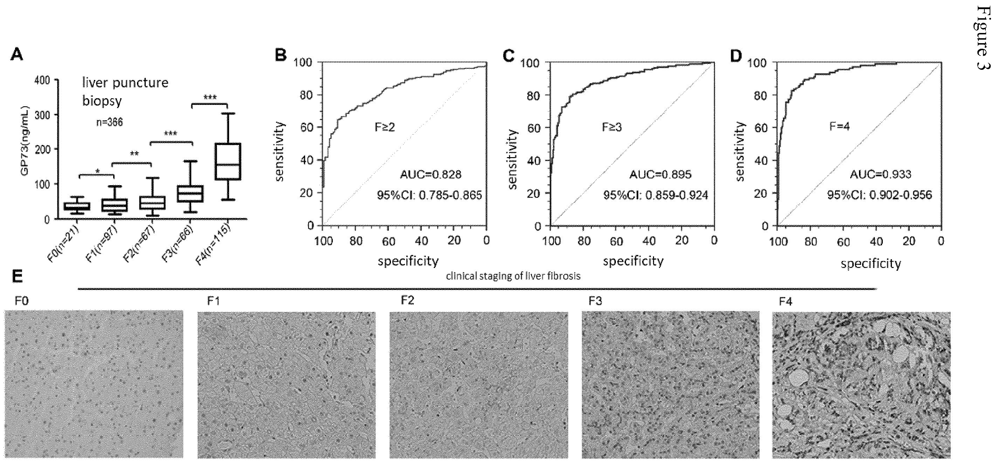

Fig. 3 shows a comparison of the diagnostic value of serum GP73 for liver fibrosis. (A) The GP73 concentrations in samples were measured in different degrees of liver fibrosis (F=0, 1, 2, 3 and 4). (B)-(D) ROC curves for different degrees of liver fibrosis. (E) Immunohistochemical images of liver in different degrees of fibrosis.

### DETAILED DESCRIPTION

The following makes further explanations to the present invention by specific implements and experimental data. Although the terminologies hereinafter are used for the purpose of clearness, they are not intended to define or limit the scope of the present disclosure.

As used in the text, the term "sensitivity" is also called "true-positive rate", which refers to the percentage of patients who are actually ill and are correctly judged as ill according to the testing criteria. The greater the sensitivity is the better. The ideal sensitivity is 100%.

As used in the text, the term "specificity" is also called "true-negative rate", which refers to the percentage of patients who are actually disease-free and are correctly judged as disease-free according to the testing criteria. The greater the specificity is the better. The ideal sensitivity is 100%.

Youden index is also called correct diagnostic index. Its value ranges between 0 ~ 1, and the closer it is to 1, the better the diagnostic accuracy. In the method of the invention, the diagnostic criterion is set at the maximum of the correct diagnostic index.

The receiver (relative) operating characteristic (ROC) reflects the balance between sensitivity and specificity. The area under the ROC curve is an important test accuracy indicator. Calculate the area under the ROC line of each test (AUCROC) for comparison. The larger the area, the greater the diagnostic value of the test.

Unless otherwise specified, the experimental methods in the following Examples are conventional methods.

Unless otherwise specified, the materials, reagents, etc. used in the following Examples can be purchased commercially.

### EXAMPLES

Example 1. Comparison of diagnostic values of serum GP73, AFP, AFP-L3 and AFP-L3 in primary hepatocellular carcinoma

### 1) Baseline data of subjects and samples

Blood samples were obtained from: 805 patients with hepatitis; 2015 patients with liver cirrhosis; 1102 patients with liver cancer, a total of 3922 patients. See Table 1 for patient's specific information.

The samples were all serum of clinically confirmed patients. The three clinical samples were provided by the People's Liberation Army No. 302 Hospital. The patients were treated in the People's Liberation Army No. 302 Hospital from December 2013 to December 2014.

### 2) Statistics

SPSS 21.0 (SPSS, Chicago, Illinois, USA) statistical software was used for statistical analysis. Descriptive methods of continuous variables are selected according to their distribution characteristics. Quantitative data approximates the normal distribution is expressed by *x̅*±S. The distribution characteristics of quantitative data with skewed distribution are described by median (M) and interquartile range. χ2 test was used for comparison among qualitative data sets, Mann-WhitnyU test was used for comparison between two independent samples, and Kruskal Wallis test was used among multiple sets of samples. The receiver operating characteristic curve (ROC curve) was established, and the value of different indicators for the diagnosis of primary hepatocellular carcinoma was compared according to the area under the curve, taking α=0.05 as testing standard.

### 3) Results

The commercial ELISA kit of Beijing Rejing Biotechnology Co. Ltd. was used to detect the level of GP73 in serum samples according to the specific experimental procedures of the kit instruction. The results showed that the level of GP73 in chronic hepatitis, cirrhosis and hepatocellular carcinoma were 45.20 ± 0.78ng/mL, 150.32 ± 1.78ng/mL and 121.32 ± 2.47ng/mL respectively, as shown in Table 1, while the level of alpha-fetoprotein AFP3 in chronic hepatitis, cirrhosis and hepatocellular carcinoma were 3.12±0.23 ng/mL, 33.51±4.64 ng/mL and 567.36± 40.71 ng/mL respectively.

**Table 1. Testing results of the subjects**

| Variate | Hepatitis (n=805) | Cirrhosis (n=2015) | Hepatocellular carcinoma | |
|---|---|---|---|---|
| | | | (n=1102) | *P* |
| | mean ± SEM | mean ± SEM | mean ± SEM | |
| Age | 44.68±0.39 | 50.58±0.25 | 52.09±0.31 | 0.000 |
| BMI(kg/m²⁾ | 24.12±0.12 | 24.14±0.09 | 24.00±0.10 | 0.785 |
| GP73(ng/mL) | 45.20±0.78 | 150.32±1.78 | 121.32±2.47 | 0.000 |
| AFP3(ng/mL) | 3.12±0.23 | 33.51±4.64 | 567.36±40.71 | 0.000 |
| ALT(U/L) | 46.47±3.14 | 93.64±5.31 | 72.23±4.68 | 0.000 |
| AST(U/L) | 35.27±1.61 | 87.47±3.61 | 79.36±4.25 | 0.000 |
| PLT(10^9/L) | 187.71±2.20 | 104.14±1.47 | 144.85±2.75 | 0.000 |
| APRI | 0.56±0.04 | 2.71±0.14 | 2.05±0.16 | 0.000 |
| FIB-4 | 1.57±0.07 | 6.92±0.21 | 4.61±0.17 | 0.000 |

Note: ALT, alanine aminotransferase; AST, glutamic oxaloacetic aminotransferase; PLT, platelet; APRI, a scoring method, the ratio index of aspartate aminotransferase (AST) and platelet (PLT); FIB-4, the evaluation index of liver disease.

### 4) Comparison of the diagnostic values of serum GP73, AFP, AFP-L3 and AFP-L3 in primary hepatocellular carcinoma

Using the pathological diagnosis as the gold standard, the ROC curve was used to calculate the sensitivity, specificity, positive predictive value, negative predictive value, false positive rate and Youden index of serum GP73, AFP, AFP-L3 and AFP-L3, and calculated the best diagnostic value. The results showed that the area under the ROC curves of serum Golgi protein 73 (GP73), alpha-fetoprotein (AFP) and alpha-fetoprotein heterogenous (AFP-L3) were 0.527 (95% CI, 0.503-0.552), 0.826 (95% CI, 0.808-0.843 P < 0.001) and 0.824 (95% CI, 0.806-0.841 P < 0.001) respectively, as shown in Fig.1 and Table 2.

**Table 2. The diagnostic values of GP73, AFP, AFP-L3(%) and AFP-L3 in hepatocellular carcinoma**

| Variate (%) | Sensitivity | Specificity | Youden index | Area under the curve | 95%CI |
|---|---|---|---|---|---|
| AFP | 68.56% | 82.45% | 0.510 | 0.826 | 0.808-0.843 |
| AFP-L3 | 67.60% | 82.65% | 0.503 | 0.824 | 0.806-0.841 |
| AFP-L3 | 54.58% | 88.02% | 0.462 | 0.677 | 0.653-0.701 |
| GP73 | 85.25% | 21.89% | 0.071 | 0.527 | 0.503-0.552 |

Example 2. The distribution characteristics of serum GF73, AFP and AFP-L3 (%) in patients with hepatitis, cirrhosis and hepatocellular carcinoma.

The subjects and statistical methods were the same as in Example 1. In the experiment, the changes of AFP, AFP-L3 (%) and GP73 in patients with hepatitis, cirrhosis and hepatocellular carcinoma were described by interquartile range. The results showed that the trend of GP73 change in hepatitis, cirrhosis and hepatocellular carcinoma was obvious, but it could not distinguish patients with hepatocellular carcinoma from those with hepatitis and cirrhosis. AFP and AFP-L3 (%) showed significant change in patients with liver cancer only, and could distinguish patients with hepatocellular carcinoma from those with hepatitis and cirrhosis, as shown in Table 3.

**Table 3. Interquartile ranges of GP73, AFP and AFP-L3 (%) in patients with hepatitis, cirrhosis and hepatocellular carcinoma**

| Variate | Disease | Interquartile range | | | | | | |
|---|---|---|---|---|---|---|---|---|
| | | 5 | 10 | 25 | 50 | 75 | 90 | 95 |
| GP73 | Hepatitis | 15.291 | 20.568 | 33.500 | 57.480 | 90.573 | 159.230 | 219.535 |
| | Cirrhosis | 26.950 | 39.260 | 72.220 | 121.600 | 179.200 | 246.400 | 277.900 |
| | Hepatocellular Carcinoma | 29.432 | 38.750 | 61.960 | 103.800 | 168.350 | 240.080 | 291.640 |
| AFP | Hepatitis | 0.896 | 1.049 | 1.490 | 2.260 | 3.600 | 10.697 | 52.828 |
| | Cirrhosis | 0.822 | 1.030 | 1.600 | 2.660 | 5.820 | 20.170 | 72.520 |
| | Hepatocellular Carcinoma | 1.740 | 2.268 | 5.145 | 40.850 | 885.850 | 1300.000 | 2245.300 |
| AFP-L3 | Hepatitis | 0.045 | 0.053 | 0.075 | 0.114 | 0.181 | 0.690 | 5.419 |
| | Cirrhosis | 0.041 | 0.052 | 0.081 | 0.135 | 0.299 | 1.665 | 7.130 |
| | Hepatocellular carcinoma | 0.087 | 0.111 | 0.256 | 3.800 | 152.600 | 1018.300 | 1100.000 |

### Example 3. Comparison of the diagnostic values of serum GP73, LSM, FIB4 and APRI in cirrhosis

The subjects and statistical methods were the same as in Example 1.

The results of this part showed that the sensitivity and specificity of GP73 in the diagnosis of cirrhosis were 83.97% and 93.54% respectively, and the area under curve was 0.927 (95% CI, 0.917-0.937) (Fig. 2). The diagnostic value of GP73 was better than that of liver stiffness measurement (LSM), FIB-4 index and platelet (PLT) ratio index APRI, as shown in Table 4, indicating that GP73 had a stronger ability to distinguish patients with cirrhosis.

### Table 4. The diagnostic values of GP73, LSM, APRI and FIB4 in cirrhosis

| Variate | Cut-off value | Sensitivity (%) | Specificity (%) | Positive predictive value% | Negative predictive value% | Youden index | Area under the curve | 95%CI | *P* |
|---|---|---|---|---|---|---|---|---|---|
| GP73 ng/ml | 75.16 | 83.97 | 93.54 | 97.02 | 69.99 | 0.775 | 0.927 | 0.917-0.937 | <0.0001 |
| LSM kPa | 11.8 | 74.19 | 86.75 | 90.63 | 66.06 | 0.609 | 0.885 | 0.866-0.901 | <0.0001 |
| FIB-4 | 2.0 | 83.17 | 82.75 | 92.35 | 66.27 | 0.659 | 0.900 | 0.888-0.910 | <0.0001 |
| APRI | 0.64 | 78.92 | 81.95 | 91.63 | 60.84 | 0.609 | 0.871 | 0.858-0.883 | <0.0001 |

### Example 4. Comparison of the diagnostic values of serum GP73 in liver fibrosis

Serum GP73 could well distinguish between significant liver fibrosis (F≥2), severe liver fibrosis (F≥3) and early cirrhosis (F=4). Serum GP73 level (Means±SE) increased with the degree of liver fibrosis F0 (n=21, 5.74%): 35.18±2.95; F1 (n=97, 26.50%): 42.73±2.57; F2 (n=67, 18.31%): 50.59±3.48; F3 (n=66, 18.03%): 79.93±5.67; F4 (n=115, 31.42%): 169.26±7.31 (P<0.001). The sensitivity, specificity and AUROC of serum GP73 in the diagnosis of F≥2, F≥3 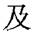 F=4 were F≥2: 64.92%, 90.68%, 0.828 (95% CI: 0.785-0.865); F≥3: 80.66%, 86.49%, 0.895 (95% CI: 0.859-0.924); F=4: 82.61%, 91.24%, 0.933 (95%CI: 0.902-0.956), as shown in Fig. 3.

The above results indicate that GP73 can be used as a potential marker for screening and discriminating liver fibrosis and cirrhosis (i. e. in non-hepatitis B virus infection) from healthy people and patients with liver fibrosis and cirrhosis, and the method of screening and diagnosing different degrees of liver fibrosis or cirrhosis (non-hepatitis B virus infection) by detecting the GP73 protein concentration in samples of the screening target is accurate and easy to operate.

## Claims

1. Use of Golgi protein 73 and/or Golgi protein 73 antibody in the in vitro/ex vivo diagnosis/prognosis of liver diseases in a sample, the liver disease being selected from a liver disease resulting from non-hepatitis B virus infection, and the diagnosis/prognosis is distinguishing between significant liver fibrosis (F ≥ 2), severe liver fibrosis (F ≥ 3) and early cirrhosis (F = 4);
wherein the sample is human serum, human plasma or human interstitial fluid previously obtained from a subject.

2. The use of claim 1, further comprising a reagent and a required instrument for detecting the Golgi protein 73 level.

3. The use of claim 1 or 2, wherein the subject is a patient with cirrhosis, liver fibrosis or healthy person.

4. Use of a kit in the in vitro/ex vivo diagnosis/prognosis of liver diseases, the kit comprising a substance of detecting Golgi protein 73 level in a sample, the liver disease being selected from a liver disease resulting from non-hepatitis B virus infection, and the diagnosis/prognosis being distinguishing between significant liver fibrosis (F ≥ 2), severe liver fibrosis (F ≥ 3) and early cirrhosis (F = 4),
wherein the sample is human serum, human plasma or human interstitial fluid,
wherein the substance of detecting Golgi protein 73 level in a sample is Golgi protein 73 and/or Golgi protein 73 antibody.

5. The use of claim 4, wherein the kit further comprises a reagent for detecting the Golgi protein 73 level.

6. The use of claim 4, wherein detection methods by the kit include enzyme-linked immunosorbent assay, chemiluminescence, electrochemiluminescence, enzymatic chemiluminescence, time-resolved fluorescence, or up conversion luminescence.

## Patentansprüche

1. Verwendung von Golgi-Protein 73 und/oder Golgi-Protein 73-Antikörper bei der In-vitro/Ex-vivo-Diagnose/Prognose von Lebererkrankungen in einer Probe, wobei die Lebererkrankung aus einer Lebererkrankung ausgewählt ist, die aus einer Infektion mit Nicht-Hepatitis-B-Virus resultiert, und die Diagnose/Prognose zwischen signifikanter Leberfibrose (F ≥ 2), schwerer Leberfibrose (F ≥ 3) und früher Zirrhose (F = 4) unterscheidet;
wobei es sich bei der Probe um menschliches Serum, menschliches Plasma oder menschliche interstitielle Flüssigkeit handelt, die zuvor von einem Individuum erhalten wurde.

2. Verwendung nach Anspruch 1, ferner umfassend ein Reagenz und ein erforderliches Instrument zum Nachweis des Golgi-Protein-73-Spiegels.

3. Verwendung nach Anspruch 1 oder 2, wobei es sich bei dem Individuum um einen Patienten mit Zirrhose, Leberfibrose oder eine gesunde Person handelt.

4. Verwendung eines Kits bei der In-vitro/Ex-vivo-Diagnose/Prognose von Lebererkrankungen, wobei das Kit eine Substanz zum Nachweisen des Golgi-Protein-73-Spiegels in einer Probe umfasst, wobei die Lebererkrankung aus einer Lebererkrankung ausgewählt ist, die aus einer Nicht-Hepatitis-B-Virusinfektion resultiert, und die Diagnose/Prognose zwischen signifikanter Leberfibrose (F ≥ 2), schwerer Leberfibrose (F ≥ 3) und früher Zirrhose (F = 4) unterscheidet, wobei es sich bei der Probe um menschliches Serum, menschliches Plasma oder menschliche interstitielle Flüssigkeit handelt,
wobei es sich bei der Substanz zum Nachweisen des Golgi-Protein 73-Spiegels in einer Probe um Golgi-Protein 73 und/oder Golgi-Protein-73-Antikörper handelt.

5. Verwendung nach Anspruch 4, wobei das Kit ferner ein Reagenz zum Nachweisen des Golgi-Protein-73-Spiegels umfasst.

6. Verwendung nach Anspruch 4, wobei Nachweisverfahren mit dem Kit ELISA (Enzyme-linked Immunosorbent Assay), Chemilumineszenz, Elektrochemilumineszenz, enzymatische Chemilumineszenz, zeitaufgelöste Fluoreszenz oder Aufwärtskonversion-Lumineszenz umfassen.

## Revendications

1. Utilisation de la protéine de Golgi 73 et/ou d'un anticorps dirigé contre la protéine de Golgi 73 dans le diagnostic/pronostic in vitro/ex vivo de maladies hépatiques dans un échantillon, la maladie hépatique étant choisie parmi une maladie hépatique résultant d'une infection non due au virus de l'hépatite B et le diagnostic/pronostic faisant la distinction entre une fibrose hépatique significative (F ≥ 2), une fibrose hépatique sévère (F ≥ 3) et une cirrhose précoce (F = 4) ;
dans laquelle l'échantillon est du sérum humain, du plasma humain ou du liquide interstitiel humain auparavant obtenu à partir d'un sujet.

2. Utilisation selon la revendication 1, comprenant en outre un réactif et un instrument requis pour la détection du taux de protéine de Golgi 73.

3. Utilisation selon la revendication 1 ou 2, dans laquelle le sujet est un patient atteint de cirrhose, de fibrose hépatique ou une personne en bonne santé.

4. Utilisation d'un kit dans le diagnostic/pronostic in vitro/ex vivo de maladies hépatiques, le kit comprenant une substance de détection du taux de protéine de Golgi 73 dans un échantillon, la maladie hépatique étant choisie parmi une maladie hépatique résultant d'une infection non due au virus de l'hépatite B et le diagnostic/pronostic faisant la distinction entre une fibrose hépatique significative (F ≥ 2), une fibrose hépatique sévère (F ≥ 3) et une cirrhose précoce (F = 4), dans laquelle l'échantillon est du sérum humain, du plasma humain ou du liquide interstitiel humain,
dans laquelle la substance de détection du taux de protéine de Golgi 73 dans un échantillon est la protéine de Golgi 73 et/ou un anticorps dirigé contre la protéine de Golgi 73.

5. Utilisation selon la revendication 4, dans laquelle le kit comprend en outre un réactif pour la détection du taux de protéine de Golgi 73.

6. Utilisation selon la revendication 4, dans laquelle les procédés de détection par le kit incluent un dosage immunoenzymatique, la chimiluminescence, l'électrochimiluminescence, la chimiluminescence enzymatique, la fluorescence résolue dans le temps ou la luminescence à conversion ascendante.
